(19) Europäisches Patentamt
European Patent Office
Office européen des brevets

(11) Veröffentlichungsnummer : **0 009 560**
**B1**

(12) **EUROPÄISCHE PATENTSCHRIFT**

(45) Veröffentlichungstag der Patentschrift : **22.09.82**

(21) Anmeldenummer : **79102712.1**

(22) Anmeldetag : **30.07.79**

(51) Int. Cl.³ : **C 07 D237/04**, C 07 D409/04, C 07 D401/04, A 61 K 31/50 // (C07D409/04, 237/00, 333/00),(C07D401/04, 237/00, 213/55)

(54) **Neue 1,4-Dihydropyridazin-3-carbonylverbindungen, Verfahren zu ihrer Herstellung, die Verbindungen zur Verwendung bei der Behandlung von Krankheiten, diese enthaltende Arzneimittel und Verfahren zur Herstellung der Arzneimittel.**

(30) Priorität : **08.08.78 DE 2834624**

(43) Veröffentlichungstag der Anmeldung : **16.04.80 (Patentblatt 80/08)**

(45) Bekanntmachung des Hinweises auf die Patenterteilung : **22.09.82 Patentblatt 82/38**

(84) Benannte Vertragsstaaten : **AT BE CH DE FR GB IT NL**

(56) Entgegenhaltungen :
**Helvetica Chimica Acta. Vol. XXXVII, Fasciculus V (1954) — No. 172 P. Schmidt und J. Druey « Zur neuen Pyridazin-Synthese. Methylpyridazine »**
**+ Seiten 1467 - 1471 +**
**Tetrahedron, Vol. 33, (1977) No. 1.-D. B. Chantegrel, D. Hartmann und S. Gelin « Synthese d'arylacetyl-3 carbethoxy ou acetyl-4 methyl-5-pyrazoles et de pyrazolo (3,4d) pyridazines »**
**+ Seiten 45 - 51**

(73) Patentinhaber : **BAYER AG**
**Zentralbereich Patente, Marken und Lizenzen**
**D-5090 Leverkusen 1, Bayerwerk (DE)**

(72) Erfinder : **Franckowiak, Gerhard, Dr.**
**Pahlkestrasse 17**
**D-5600 Wuppertal (DE)**
Erfinder : **Bossert, Friedrich, Dr.**
**Claudiusweg 7**
**D-5600 Wuppertal 1 (DE)**
Erfinder : **Heise, Arend, Dr.**
**Möbeck 50**
**D-5600 Wuppertal 11 (DE)**
Erfinder : **Towart, Robertson, Dr.**
**Claudiusweg 9**
**D-5600 Wuppertal 1 (DE)**

**EP 0 009 560 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Neue 1,4-Dihydropyridazin-3-carbonylverbindungen, Verfahren zu ihrer Herstellung, die Verbindungen zur Verwendung bei der Behandlung von Krankheiten, diese enthaltende Arzneimittel und Verfahren zur Herstellung der Arzneimittel

Die vorliegende Erfindung betrifft neue 1,4-Dihydropyridazin-3-carbonylverbindungen, Verfahren zu ihrer Herstellung die Verbindungen zur Verwendung bei der Behandlung von Krankheiten, diese enthaltende Arzneimittel insbesondere kreislaufbeeinflussende, spasmolytisch und antiinflammatorisch wirksame Mittel und Verfahren zu ihrer Herstellung.

Es ist bereits bekanntgeworden, daß man 1,4-Dihydropyridazine erhält, wenn man substituierte 1,4-Dicarbonylverbindungen mit Hydrazinhydrat umsetzt (vgl. W. Borsche und M. Spannagel, Liebigs Ann. Chem. *331,* 300 (1904)). Der Darstellbarkeit solcher 1.4-Dicarbonylverbindungen sind erfahrungsgemäß jedoch Grenzen gesetzt, wenn mehrere Carbonylfunktionen durch Ester- und Acylsubstituenten in das Molekül eingeführt werden. Überraschenderweise gelang nun die Umsetzung solcher Verbindungen als anzunehmende Reaktionszwischenstufen in situ mit Hydrazin bzw. Hydrazinhydrat zu den erfindungsgemäßen 1,4-Dihydropyridazin-3-carbonylverbindungen.

Die vorliegende Erfindung betrifft neue 1,4-Dihydropyridazin-3-carbonylverbindungen der Formel (I)

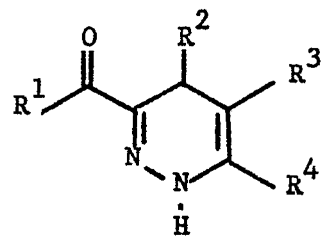

(I)

in welcher

$R^1$ für die Gruppen $OR^5$ oder

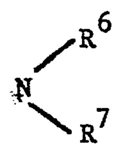

steht, wobei

$R^5$ für Wasserstoff, Allyl, geradkettiges oder verzweigtes Alkyl oder Cycloalkyl mit jeweils bis zu 8 Kohlenstoffatomen, steht, wobei die Alkylkette gegebenenfalls durch 1 Sauerstoff unterbrochen ist oder gegebenenfalls durch eine Aminogruppe mit zwei gleichen oder verschiedenen Substituenten aus der Gruppe Methyl oder Benzyl substituiert ist,

$R^6$ und $R^7$ verschieden sind und jeweils für Wasserstoff, einen Methyl- oder Benzyl- oder einen gegebenenfalls einfach durch Halogen substituierten Phenylrest stehen,

$R^2$ für einen Phenylrest, der gegebenenfalls durch 1 oder 2 gleiche oder verschiedene Substituenten aus der Gruppe Nitro, Halogen, Cyano, Trifluormethyl, Trifluormethoxy, Alkyl oder Alkoxy mit jeweils 1 bis 2 Kohlenstoffatomen substituiert ist oder für einen Thienyl- oder Pyridylrest steht,

$R^3$ für Wasserstoff oder die Gruppe $COOR^8$ steht, wobei $R^8$ die Bedeutung von $R^5$ besitzt aber nicht mit $R^5$ identisch sein muß und

$R^4$ für einen Methyl-, Ethyl- oder Phenylrest steht.

Die Erfindung betrifft auch 1,4-Dihydro-6-methyl-4-(3-nitrophenyl)-pyridazin-3,5-dicarbonsäure-5-ethyl-3-l-menthylester (gemäß Beispiel 35).

Die erfindungsgemäßen Verbindungen stellen eine neuartige Stoffklasse zur Kreislaufbehandlung, zur Behandlung des Magen-Darm-Traktes, des Urogenital-Traktes und des Respirationssystems dar und verfügen über eine antiinflammatorische Wirkung ; Ein solcher Wirkungsspektrum war aufgrund des Standes der Technik nicht vorhersehbar.

Die neuen erfindungsgemäßen 1,4-Dihydropyridazine besitzen somit wertvolle pharmakologische Eingenschaften. Aufgrund ihrer kreislaufbeeinflussenden spasmolytischen und antiinflammatorischen Wirkung können sie als antihypertensive Mittel, als Vasodilatatoren, als Coronartherapeutika, als Spasmolytika und als Entzündungshemmer Verwendung finden.

Es wurde nun gefunden, daß man die neuen 1,4-Dihydropyridazine der Formel (I) erhält, wenn man Nitronsäuren der Formel (II)

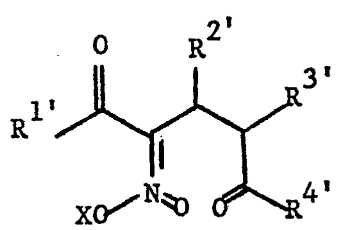

(II)

in welcher

$R^{1'}$ für die Gruppe $OR^{5'}$ steht, wobei $R^{5'}$ für Allyl, geradkettiges oder verzweigtes Alkyl oder Cycloalkyl mit jeweils bis zu 8 Kohlenstoffatomen steht, wobei die Alkylkette gegebenenfalls durch 1 Sauerstoff unterbrochen ist,

$R^{3'}$ für Wasserstoff oder die Gruppe $COR^{8'}$, wobei $R^{8'}$ die oben für $R^8$ angegebene Bedeutung besitzt,

$R^{2'}$ und $R^{4'}$ die oben für $R^2$ und $R^4$ angegebene Bedeutung besitzen, und

X für ein Kation wie Wasserstoff-, Alkali-, Ammonium- oder Alkylamoniumion steht, zunächst mit Ozon (vergl. J. Org. Chem. *39* (1974), S. 259 f) und dann mit Hydrazin bzw. Hydrazinhydrat in inerten organischen Lösungsmitteln umsetzt und gegebenenfalls die so erhaltenen Verbindungen nach allgemein bekannten Methoden der Verseifung, Veresterung, Umesterung oder Amidierung zu weiteren erfindungsgemäßen Verbindungen der allgemeinen Formel I bzw. entsprechend zu 1,4-Dihydro-6-methyl-4-(3-nitrophenyl)-pyridazin-3,5-dicarbonsäure-5-ethyl-3-l-menthylester derivatisiert.

Verwendet man das Natriumsalz des 2-Acetyl-4-aci-nitro-3-(2-nitrophenyl)-glutarsäurediethylesters, Ozon und Hydrazin als Ausgangstoffe, so kann der Reaktionsablauf durch das folgende Formelschema beispielhaft wiedergegeben werden

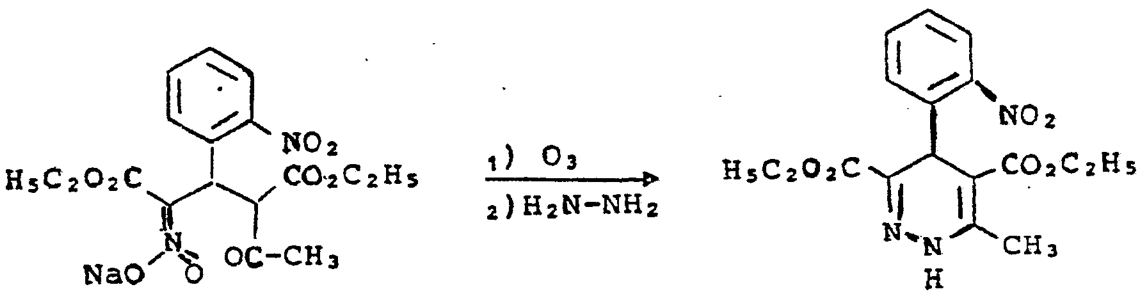


Gemäß der angegebenen Verfahrensweise wird eine Nitronsäure der Formel (II) (X=H) oder ein Salz hiervon zunächst mit Ozon und im folgenden mit Hydrazin zu einem 1.4-Dihydropyridazinderivat der Formel I umgesetzt.

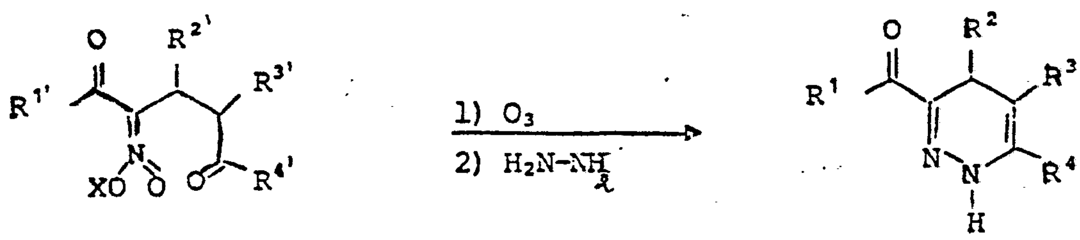


(II) (I)

Die erfindungsgemäß als Ausgangsstoffe verwendeten Nitronsäuren und deren Salze können z.B. durch basenkatalysierte Michael-Addition von Nitroessigsäureestern an Ylidencarbonylverbindungen dargestellt werden (vgl. A. Dornow und A. Frese, Ann. Chem. 578, 122 (1952)).

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen − 120 °C und + 120 °C, vorzugsweise im Bereich zwischen − 80 und + 20 °C.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird 1 Mol der aci-Verbindung der Formel (II) zunächst bei tiefer Temperatur in einem geeigneten Lösungsmittel ozonisiert und dann mit einem Mol Hydrazin bei Raumtemperatur zur Reaktion gebracht.

Die Isolierung und Reinigung der erfindungsgemäßen Substanzen erfolgt vorzugsweise derart, daß man gegebenenfalls nach Abtrennung unlöslicher Stoffe das Lösungsmittel im Vakuum abdestilliert und das gegebenenfalls erst nach Chromatographie kristallin erhaltene Produkt aus einem geeigneten Lösungsmittel umkristallisiert.

In Ergänzung zu dem zuvor beschriebenen Verfahren können durch literaturbekannte Methoden der Verseifung, Veresterung oder Umesterung oder der Amidierung (vgl. Organikum, VEB Deutscher Verlag der Wissenschaften, Berlin 1967) entsprechend weitere erfindungsgemäße Derivate aus den nach dem erfindungsgemäßen Verfahren zugänglichen 1,4-Dihydropyridazin-mono- und -dicarbonsäureestern

hergestellt werden.

Hierzu seien folgende Verfahren aufgeführt :

a) Verseifung

1,4-Dihydropyridazin-3-carbonsäureester (Formel I, $R^1 = OR^5$) und -3,5-dicarbonsäureester (Formel I, $R^1 = OR^5$, $R^3 = COOR^8$) lassen sich mit einem Äquivalent Alkali selektiv zu den 1,4-Dihydropyridazin-3-carbonsäuren (Formel I, $R^1 = OH$) verseifen. Die Verseifung mit einem Überschuß Alkali führt zu den 1,4-Dihydropyridazin-3,5-dicarbonsäuren (Formel I, $R^1 = OH$, $R^3 = COOH$).

b) Veresterung

1,4-Dihydropyridazin-3-carbonsäuren (Formel I, $R^1 = OH$), 1,4-Dihydropyridazin-3,5-dicarbonsäurehalbester (Formel I, $R^1 = OH$, $R^3 = COOR^8$ oder $R^1 = OR^5$, $R^3 = COOH$) und 1,4-Dihydropyridazin-3,5-dicarbonsäuren (Formel I, $R^1 = OH$, $R^3 = COOH$) lassen sich mit Alkoholen, Diazoalkanen, Dialkylsulfaten oder Alkylhalogeniden zu den entsprechenden Estern (Formel I, $R^1 = OR^5$ oder $R^1 = OR^5$, $R^5 = COOR^8$) umsetzen.

c) Umesterung

1,4-Dihydropyridazin-3-carbonsäureester (Formel I, $R^1 = OR^5$) und -3,5-dicarbonsäureester (Formel I, $R^1 = OR^5$, $R^3 = COOR^8$) lassen sich im Alkoholen säure- oder basenkatalysiert zu den entsprechenden Estern umsetzen.

d) Amidierung

1,4-Dihydropyridazin-3-carbonsäuren (Formel I, $R^1 = OH$), 1,4-Dihydropyridazin-3-carbonsäureester (Formel I, $R^1 = OR^5$), 1,4-Dihydropyridazin-3,5-dicarbonsäurehalbester (Formel I, $R^1 = OH$, $R^3 = COOR^8$ oder $R^1 = OR^5$, $R^3 = COOH$), 1,4-Dihydropyridazin-3,5-dicarbonsäuren (Formel I, $R^1 = OH$, $R^3 = COOH$) und 1,4-Dihydropyridazin-3,5-dicarbonsäureester (Formel I ; $R^1 = OR^5$, $R^3 = COOR^8$) lassen sich in geeigneten Lösungsmittel mit primären oder sekundären aliphatischen oder aromatischen Aminen in die entsprechenden Amide (Formel I, $R^1 = NR^6R^7$ oder $R^3 = COOR^8$ oder $R^1 = OR^5$, $R^3 = CONR^6R^7$ oder $R^1 = NR^6R^7$, $R^3 = CONR^6R^7$) überführen.

Die vorstehenden Herstellungsverfahren sind lediglich zur Verdeutlichung angegeben, und die Herstellung der Verbindung der Formel I ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen existieren in stereoisomeren Formen, die sich entweder wie Bild und Spiegelbild (Enantiomere) oder die sich nicht wie Bild und Spiegelbild (Diastereomer) verhalten. Sowohl die Antipoden als auch die Racemformen als die Diastereomerengemische sind Gegenstand der vorliegenden Erfindung. Die Racemformen lassen sich ebenso wie die Diastereomeren in bekannter Weise in die stereoiosmer einheitlichen Bestandteile trennen (vgl. z.B. E. L. Eliel, Stereochemistry of Carbon Compounds, McGraw Hill, 1962).

Die neuen Verbindungen haben ein breites und vielseitiges pharmakologisches Wirkungsspektrum.

Im einzelnen konnten im Tierexperiment folgende Hauptwirkungen nachgewiesen werden :

1) Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen Nitrit-ähnlichen herzentlastenden Effekt verstärkt. Sie beeinflussen bzw. verändern den Herzstoffwechsel im Sinne einer Energieersparnis.

2) Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3) Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren.

4) Die Verbindungen senken den Blutdruck von normotonen und hypertonen Tieren und können somit als antihypertensive Mittel verwendet werden.

5) Die Verbindungen haben stark muskulär-spasmolytische Wirkungen, die an der glatten Muskulatur des Magen-Darmtraktes, des Urogenitaltraktes und des Respirationssystems deutlich werden.

6) Die Verbindungen besitzen eine antiinflammatorische Wirkung.

Die neuen Wirkstoffe können in bekannter Weise in die üblichen Formulierungen übergeführt werden, wie Tabletten, Kapseln, Dragees, Pillen, Granulate, Aerosole, Sirups, Emulsionen, Suspensionen und Lösungen unter Werwendung inerter, nichttoxischer pharmazeutisch geeigneter Trägerstoffe oder Lösungsmittel. Hierbei soll die therapeutisch wirksame Verbindung jeweils in einer Konzentration von 0,5 bis 90 Gewichtsprozent der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt :

Wasser, nichttoxische organische Lösungsmittel, wie Paraffine (z.B. Erdölfraktionen), pflanzliche Oele (z.B. Erdnuß-/Sesamöl), Alkohole (z.B. Aethylalkohol, Glycerin), Glykole (z.B. Propylenglykol,

Polyäthylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nichtionogene anionische Emulgatoren (z.B. Polyoxyäthylen-Fettsäure-Ester, Polyoxyäthylen-Fettalkohol-Aether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in überlicher Weise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen wie Stärke, vorzugsweise Kartoffelstärke, Gelatine und dergleichen enthalten.

Weiterhin können Gleitmittel wie Magnesiumstearat, Natriumlaurylsulfat und Talkum zum Tablettieren mitverwendet werden. Im Falle wäßrigen Suspension und/oder Elixieren, die für orale Anwendung gedacht sind, können die Wirkstoffe außer mit den obengenannten Hilfsstoffen mit verschiedenen Geschmacksaufbesserern oder Farbstoffen versetzt werden. Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von 0,001 bis 10 mg/kg, vorzugsweise 0,05 bis 5 mg/kg Körpergewicht pro Tag zur Erzielung wirksamer Ergebnisse zu verabreichen, und bei oraler Applikation beträgt die Dosierung 0,01 bis 20 mg/kg, vorzugsweise 0,1 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Herstellungsbeispiele

### Beispiel 1

1.4-Dihydro-6-methyl-4-phenyl-pyridazin-3.5-dicarbonsäuredimethylester

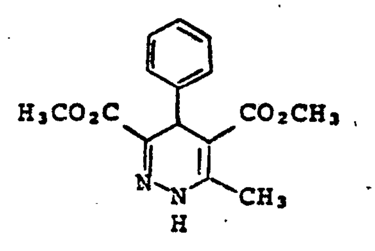

In eine Lösung von 69,0 g (0,2 Mol) des Natriumsalzes vom 2-Acetyl-4-aci-nitro-3-phenyl-glutarsäure-dimethylester in 300 ml absol. Methanol wird bei −78 °C trockenes Ozon im Überschuß bis zur Blaufärbung der Lösung eingeleitet. Nach 30 Min. wird durch Einleiten von trockenem Stickstoff das Ozon entfernt und der Ansatz unter Stickstoff auf −20° aufgewärmt. 10 g (0,2 Mol) Hydrazinhydrat in 50 ml absol. Methanol werden zugetropft und der Ansatz 12 h bei Raumtemperatur gerührt. Zur Aufarbeitung wird das Methanol i.Vak. abdestilliert, der Rückstand in Chloroform aufgenommen und vom Unlöslichen abfiltriert. Das Chloroform wird abdestilliert ; aus dem Rückstand kristallisiert das Produkt nach Zugabe von wenig Isopropanol langsam aus. Schmp. : 187°.

Ausbeute : 47 % d. Th.

### Beispiel 2

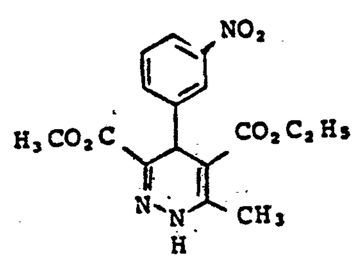

Analog Beispiel 1 wurde durch Umsetzung von 0,2 Mol des Natriumsalzes vom 2-Acetyl-4-aci-nitro-3-(3-nitrophenyl)-glutarsäure-1-ethyl-5-methylester mit überschüssigem Ozon und 0,2 Mol Hydrazinhydrat 1.4-Dihydro-6-methyl-4-(3-nitrophenyl)-pyridazin-3.5-dicarbonsäure-5-ethyl-3-methylester vom Schmp. 176° (aus Isopropanol nach Chromatographie über Kieselgel mit $CHCl_3$/MeOH) erhalten.

Ausbeute : 43 % d. Th.

Beispiel 3

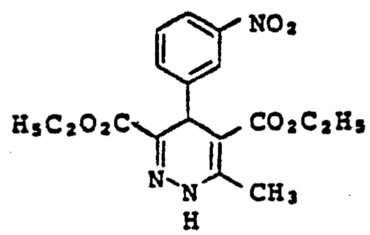

Analog Beispiel 1 wurde durch Umsetzung von 0,2 Mol des Natriumsalzes vom 2-Acetyl-4-aci-nitro-3-(3-nitrophenyl)-glutarsäurediethylester mit überschüssigem Ozon und 0,2 Mol Hydrazinhydrat 1.4-Dihydro-6-methyl-4-(3-nitrophenyl)-pyridazin-3,5-dicarbonsäurediethylester vom Schmp. 147° (Methanol) erhalten.

Ausbeute : 72,8 % d. Th.

Beispiel 4

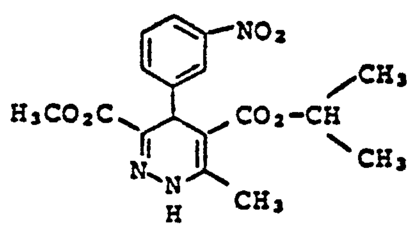

Analog Beispiel 1 wurde durch Umsetzung von 0,2 Mol des Natriumsalzes vom 2-Acetyl-4-aci-nitro-3-(3-nitrophenyl)-glutarsäure-1-isopropyl-5-methylester mit überschüssigem Ozon und 0,2 Mol Hydrazinhydrat 1,4-Dihydro-6-methyl-4-(3-nitrophenyl)-pyridazin-3,5-dicarbonsäure-5-isopropyl-3-methylester vom Schmelzpunkt 193 °C (aus Isopropanol nach Kieselgelchromatographie) erhalten.

Beispiel 5

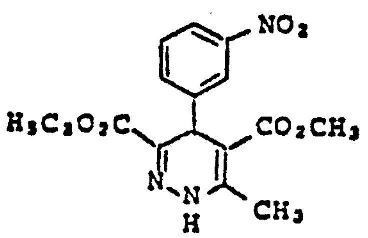

Analog Beispiel 1 wurde durch Umsetzung von 0,2 Mol des Natriumsalzes vom 2-Acetyl-4-aci-nitro-3-(3-nitrophenyl)-glutarsäure-5-ethyl-1-methylester mit überschüssigem Ozon und 0,2 Mol Hydrazinhydrat 1,4-Dihydro-6-methyl-4-(3-nitrophenyl)-pyridazin-3,5-dicarbonsäure-3-ethyl-5-methylester vom Schmelzpunkt 174° (aus Isopropanol) erhalten.

Ausbeute : 55 % d. Th.

Beispiel 6

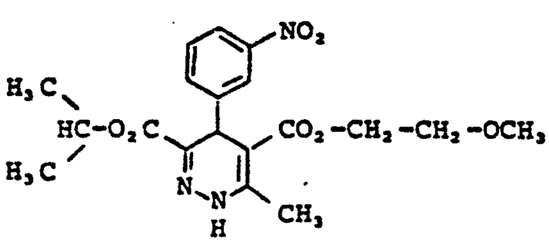

Analog Beispiel 1 wurde durch Umsetzung von 0,2 Mol des Natriumsalzes vom 2-Acetyl-4-aci-nitro-3-(3-nitrophenyl)-glutarsäure-1-(β-methoxyethyl)-5-isopropylester mit überschüssigem Ozon und 0,2 Mol Hydrazinhydrat 1.4-Dihydro-6-methyl-4-(3-nitrophenyl)-pyridazin-3,5-dicarbonsäure-3-isopropyl-5-(β-methoxyethyl)-ester vom Schmp. 146-148° (aus Isopropanol nach Kieselgelchromatographie) erhalten.
Ausbeute : 19 % d. Th.

Beispiel 7

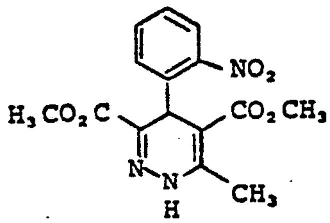

Analog Beispiel 1 wurde durch Umsetzung von 0,2 Mol des Natriumsalzes vom 2-Acetyl-4-aci-nitro-3-(2-nitrophenyl)-glutarsäure-dimethylester mit überschüssigem Ozon und 0,2 Mol Hydrazinhydrat 1.4-Dihydro-6-methyl-4-(2-nitrophenyl)-pyridazin-3.5-dicarbonsäuredimethylester vom Schmp. 203-205° (aus Ethanol nach Kieselgelchromatographie) erhalten.
Ausbeute : 42 % d. Th.

Beispiel 8

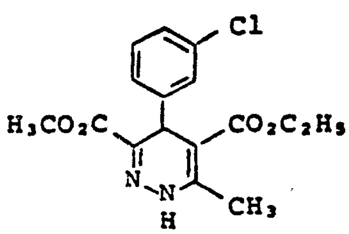

Analog Beispiel 1 wurde durch Umsetzung von 0,2 Mol des Natriumsatzes vom 2-Acetyl-4-aci-nitro-3-(3-chlorphenyl)-glutarsäure-1-ethyl-5-methylester mit überschüssigem Ozon und 0,2 Mol Hydrazinhydrat 4-(3-Chlorphenyl)-1.4-dihydro-6-methyl-pyridazin-3.5-dicarbonsäure-5-ethyl-3-methyl-ester vom Schmp. 139° (aus Methanol nach Kieselgelchromatographie) erhalten.
Ausbeute : 51 % d. Th.

Beispiel 9

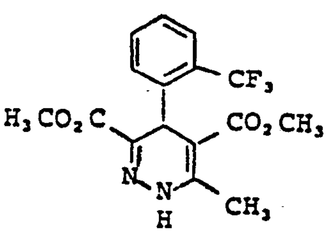

Analog Beispiel 1 wurde durch Umsetzung von 0,1 Mol des Natriumsalzes vom 2-Acetyl-4-aci-nitro-3-(2-trifluormethyl-phenyl)-glutarsäuredimethylester mit überschüssigem Ozon und 0,1 Mol Hydrazinhydrat 1.4-Dihydro-6-methyl-4-(2-trifluormethylphenyl)-pyridazin-3.5-dicarbonsäuredimethylester vom Schmp. 205° (Methanol) erhalten.
Ausbeute : 51 % d. Th.

Beispiel 10

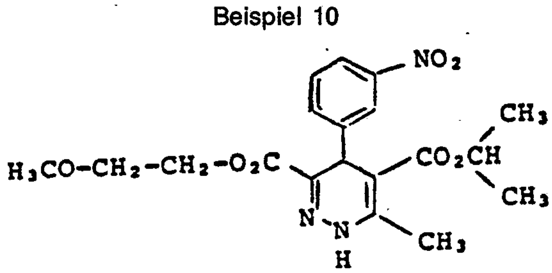

Analog Beispiel 1 wurde durch Umsetzung von 0,1 Mol des Natriumsalzes vom 2-Acetyl-4-aci-nitro-3-(3-nitrophenyl)-glutarsäure-1-isopropyl-5-(β-methoxyethyl)-ester mit überschüssigem Ozon und 0,1 Mol Hydrazinhydrat 1.4-Dihydro-6-methyl-4-(3-nitrophenyl)-pyridazin-3.5-dicarbonsäure-5-isopropyl-3-(β-methoxyethyl)-ester vom Schmp. 165° (Isopropanol) erhalten.

Ausbeute : 42 % d. Th.

Beispiel 11

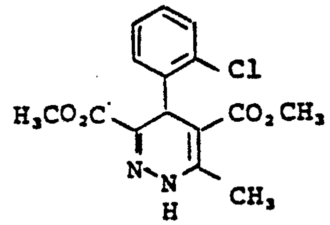

Analog Beispiel 1 wurde durch Umsetzung von 0,2 Mol des Natriumsalzes vom 2-Acetyl-4-aci-nitro-3-(2-chlorphenyl)-glutarsäuredimethylester mit überschüssigem Ozon und 0,2 Mol Hydrazinhydrat 4-(2-Chlorphenyl)-1.4-dihydro-6-methylpyridazin-3.5-dicarbonsäuredimethylester vom Schmp. 177° (Ethanol) erhalten.

Ausbeute : 62 % d. Th.

Beispiel 12

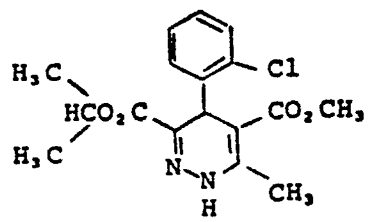

Analog Beispiel 1 wurde durch Umsetzung von 0,1 Mol des Natriumsalzes vom 2-Acetyl-4-aci-nitro-3-(2-chlorphenyl)-glutarsäure-1-methyl-5-isopropylester mit überschüssigem Ozon und 0,1 Mol Hydrazinhydrat 4-(2-Chlorphenyl)-1.4-dihydro-6-methyl-pyridazin-3.5-dicarbonsäure-3-isopropyl-5-methylester Vom Schmp. 166° (aus Ethanol nach Säulenchromatographie) erhalten.

Ausbeute : 38 % d. Th.

Beispiel 13

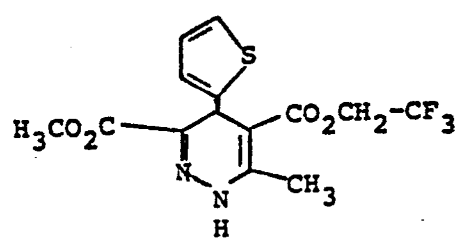

Analog Beispiel 1 wurde durch Umsetzung von 0,2 Mol des Natriumsalzes vom 2-Acetyl-4-aci-nitro-3-thien-2-yl-glutarsäure-5-methyl-1-(β-trifluorethyl)-ester mit 0,2 Mol Ozon und 0,2 Hydrazinhydrat 1,4-Dihydro-6-methyl-4-thien-2-yl-pyridazin-3,5-dicarbonsäure-3-methyl-5-(β-trifluorethyl)ester vom Schmp. 151 °C (aus Ethanol nach Säulenchromatographie) erhalten.

Ausbeute : 29 % d. Th.

Beispiel 14

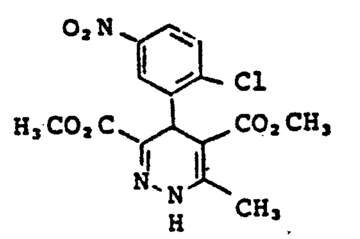

Analog Beispiel 1 wurde durch Umsetzung von 0,2 Mol des Natriumsalzes vom 2-Acetyl-4-aci-nitro-3(2-chlor-5-nitrophenyl)-glutarsäuredimethylester mit überschüssigem Ozon und 0,2 Mol Hydrazinhydrat 4-(2-Chlor-5-nitrophenyl)-1.4-dihydro-6-methyl-pyridazin-3.5-dicarbonsäuredimethylester vom Schmp. 193-4° (Methanol) erhalten.

Ausbeute : 68 % d. Th.

Beispiel 15

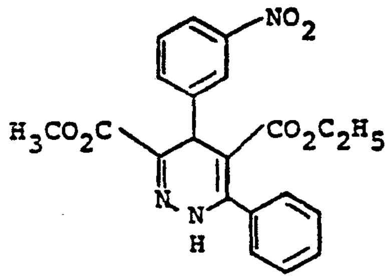

Analog Beispiel 1 wurde durch Umsetzung von 0,2 Mol des Natriumsalzes von 4-aci-Nitro-2-benzoyl-3-(3-nitro-phenylglutarsäure-1-ethyl-5-methylester mit überschüssigem Ozon und 0,2 Mol Hydrazinhydrat 1,4-Dihydro-4-(3-nitrophenyl)-6-phenyl-pyridazin-3,5-dicarbonsäure-5-ethyl-4-methylester vom Schmp. 187 °C (Methanol) erhalten.

Ausbeute : 37 % d. Th.

Beispiel 16

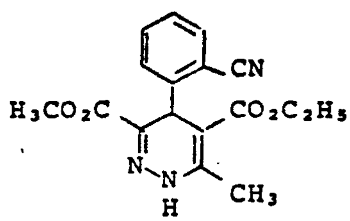

Analog Beispiel 1 wurde durch Umsetzung von 0,2 Mol des Natriumsalzes vom 2-Acetyl-4-aci-nitro-3-(2-cyanophenyl)-glutarsäure-2-ethyl-5-methylester mit überschüssigem Ozon und 0,2 Mol Hydrazinhydrat 4-(2-Cyanophenyl)-1.4-dihydro-6-methylpyridazin-3.5-dicarbonsäure-5-ethyl-3-methylester vom Schmp. 199° (Ethanol) erhalten.

Ausbeute : 57 % d. Th.

Beispiel 17

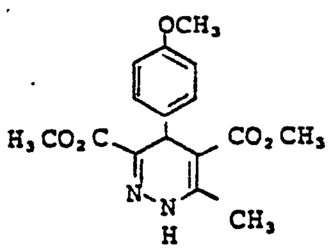

Analog Beispiel 1 wurde durch Umsetzung von 0,1 Mol des Natriumsalzes vom 2-Acetyl-4-aci-nitro-3-(4-methoxyphenyl)-glutarsäuredimethylester mit überschüssigem Ozon und 0,1 Mol Hydrazinhydrat 1.4-Dihydro-4-(4-methoxyphenyl)-6-methylpyridazin-3.5-dicarbonsäuredimethylester vom Schmp. 154° (Isopropanol) erhalten.

Ausbeute : 51 % d. Th.

Beispiel 18

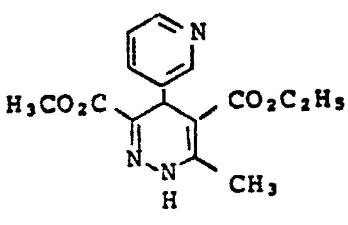

Analog Beispiel 1 wurde durch Umsetzung von 0,2 Mol des Natriumsalzes vom 2-Acetyl-4-aci-nitro-3-pyrid-3-yl-glutarsäure-1-ethyl-5-methylester mit 0,2 Mol Ozon und 0,2 Mol Hydrazinhydrat 1.4-Dihydro-6-methyl-4-pyrid-3-yl-pyridazin-3.5-dicarbonsäure-5-ethyl-3-methylester vom Schmp. 179° (aus Isopropanol nach Säulenchromatographie) erhalten.

Ausbeute : 38 % d. Th.

Beispiel 19

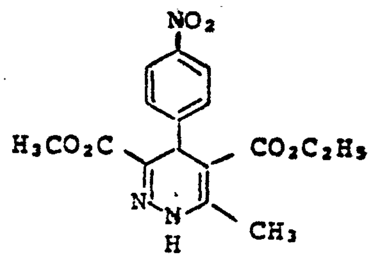

Analog Beispiel 1 wurde durch Umsetzung von 0,2 Mol des Natriumsalzes vom 2-Acetyl-4-aci-nitro-3-(4-nitrophenyl)-glutarsäure-1-ethyl-5-methylester mit überschüssigem Ozon und 0,2 Mol Hydrazinhydrat 1.4-Dihydro-6-methyl-4-(4-nitrophenyl)-pyridazin-3.5-dicarbonsäure-5-ethyl-3-methylester vom Schmp. 205-6° (Methanol) erhalten.

Ausbeute : 61 % d. Th.

Beispiel 20

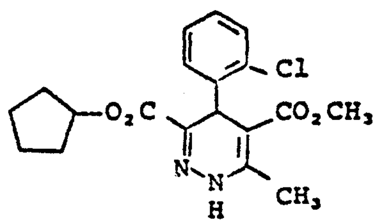

Analog Beispiel 1 wurde durch Umsetzung von 0,1 Mol des Natriumsalzes vom 2-Acetyl-4-aci-nitro-3-(2-chlorphenyl)-glutarsäure-5-cyclopentyl-1-methylester mit überschüssigem Ozon und 0,1 Mol Hydrazinhydrat 4-(2-Chlorphenyl)-1.4-dihydro-6-methyl-pyridazin-3.5-dicarbonsäure-3-cyclopentyl-5-methylester vom Schmp. 152° (Isopropanol) erhalten.

Ausbeute : 46 % d. Th.

Beispiel 21

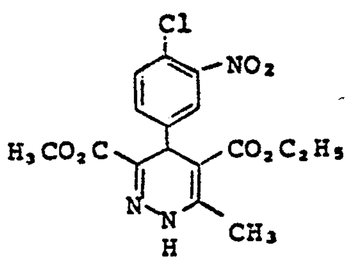

Analog Beispiel 1 wurde durch Umsetzung von 0,2 Mol des Natriumsalzes vom 2-Acetyl-4-aci-nitro-3-(4-chlor-3-nitro-phenyl)-glutarsäure-1-ethyl-5-methylester mit überschüssigem Ozon und 0,2 Mol Hydrazin 4-(4-Chlor-3-nitrophenyl)-1.4-dihydro-6-methylpyridazin-3.5-dicarbonsäure-5-ethyl-3-methylester vom Schmp. 169° (Methanol) erhalten.

Ausbeute : 58 % d. Th.

Beispiel 22

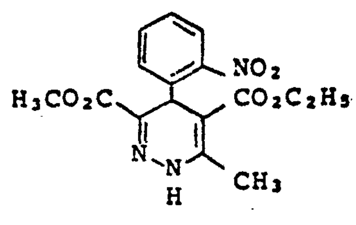

Analog Beispiel 1 wurde durch Umsetzung von 0,2 Mol des Natriumsalzes vom 2-Acetyl-4-aci-nitro-3-(2-nitrophenyl)-glutarsäure-1-ethyl-5-methylester mit überschüssigem Ozon und 0,2 Mol Hydrazinhydrat 1.4-Dihydro-6-methyl-4-(2-nitrophenyl)-pyridazin-3.5-dicarbonsäure-5-ethyl-3-methylester vom Schmp. 168-9° (aus Isopropanol nach Kieselgelchromatographie) erhalten.
Ausbeute : 39 % d. Th.

Beispiel 23

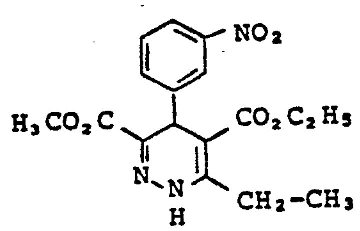

Analog Beispiel 1 wurde durch Umsetzung von 0,1 Mol des Natriumsalzes vom 4-aci-nitro-3-(3-nitrophenyl)-1-propionylglutarsäure-1-ethyl-5-methylester mit überschüssigem Ozon und 0,1 Mol Hydrazinhydrat 6-Ethyl-1.4-dihydro-4-(3-nitrophenyl)-pyridazin-3.5-dicarbonsäure-5-ethyl-3-methylester vom Schmp. 139° (Ethanol) erhalten.
Ausbeute : 55 % d. Th.

Beispiel 24

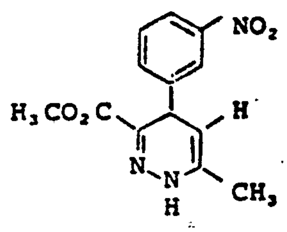

Analog Beispiel 1 wurde durch Umsetzung von 0,1 Mol des Natriumsalzes vom 2-aci-nitro-5-oxo-3-(3-nitrophenyl)-hexansäuremethylester mit überschüssigem Ozon und 0,1 Mol hydrazinhydrat 1.4-Dihydro-6-methyl-4-(3-nitrophenyl)-pyridazin-3-carbonsäuremethylester vom Schmp. 149° (aus Ethanol nach Kieselgelchromatographie) erhalten.
Ausbeute : 72 % d. Th.

Beispiel 25

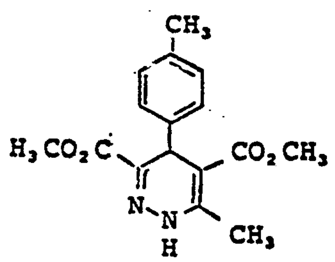

Analog Beispiel 1 wurde durch Umsetzung von 0,2 Mol des Natriumsalzes vom 2-Acetyl-4-aci-nitro-3-tolu-4-yl-glutarsäuredimethylester mit überschüssigem Ozon und 0,2 Mol Hydrazinhydrat 1,4-Dihydro-6-methyl-4-tolu-4-yl-pyridazin-3,5-dicarbonsäuredimethylester vom Schmp. 180 °C (aus Ethanol nach Kieselgelchromatographie) erhalten.
Ausbeute : 62 % d. Th.

Beispiel 26

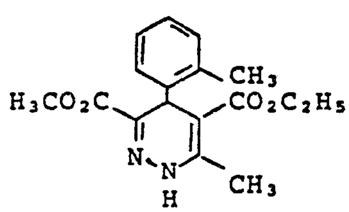

Analog Beispiel 1 wurde durch Umsetzung von 0,2 Mol des Natriumsalzes vom 2-Acetyl-4-aci-nitro-3-tolu-2-yl-glutarsäure-3-ethyl-5-methylester mit überschüssigem Ozon und 0,2 Mol Hydrazinhydrat 1.4-Dihydro-6-methyl-4-tolu-2-yl-pyridazin-3.5-dicarbonsäure-5-ethyl-3-methylester vom Schmp. 165° (aus Ethanol nach Kieselgelchromatographie) erhalten.

Ausbeute : 49 % d. Th.

Beispiel 27

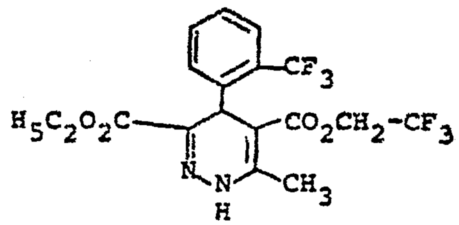

Analog Beispiel 1 wurde durch Umsetzung von 0,2 Mol des Natriumsalzes von 2-Acetyl-4-aci-nitro-3-(2-trifluormethylphenyl)-glutarsäure-5-ethyl-1-(β-trifluorethyl)-ester mit überschüssigem Ozon und 0,2 Mol Hydrazinhydrat 1,4-Dihydro-6-methyl-4-(2-trifluormethylphenyl)-pyridazin-3,5-dicarbonsäure-3-ethyl-5-(β-trifluorethyl)-ester vom Schmp. 197 °C (aus Isopropanol nach Kieselgelchromatographie) erhalten.

Ausbeute : 41 % d. Th.

Beispiel 28

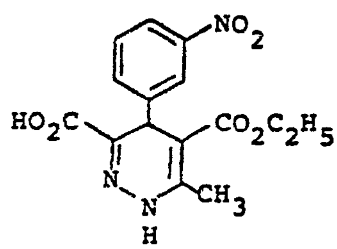

0,1 Mol 1,4-Dihydro-6-methyl-4-(3-nitrophenyl)-pyridazin-3,5-dicarbonsäurediethylester werden mit 0,15 Mol KOH in 150 ml 50 proz. wäßrigen Ethanol 8 h am Rückflußkühler erhitzt. Das Ethanol wurde i. Vak. abdestilliert, der wäßrige Rückstand mit Chloroform extrahiert, auf pH 1 angesäuert und das Produkt mit Chloroform extrahiert. Die org. Phase wurde eingeengt, aus dem Rückstand kristallisierte mit wenig Ethanol 1,4-Dihydro-6-methyl-4-(3-nitrophenyl)-pyridazin-3,5-dicarbonsäure-5-ethylester vom Schmelzpunkt 205 °C (Zers.)

Ausbeute : 91 % d. Th.

Beispiel 29

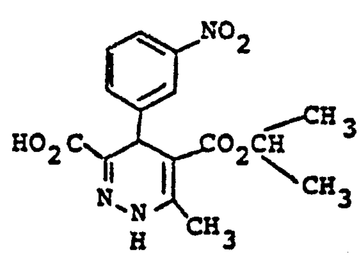

Analog Beispiel 28 wurde 0,1 Mol 1,4-Dihydro-6-methyl-4-(3-nitrophenyl)-pyridazin-3,5-dicarbonsäure-5-isopropyl-3-methylester mit 0,105 Mol KOH zu 1,4-Dihydro-6-methyl-4-(3-nitrophenyl)-pyridazin-3,5-dicarbonsäure-5-isopropylester verseift. Schmp. 195 °C (Ethanol).

Ausbeute : 92 % d. Th.

Beispiel 30

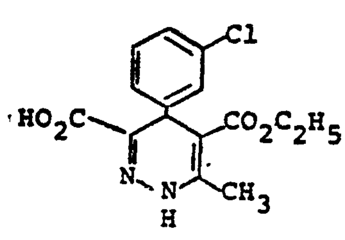

Analog Beispiel 28 wurde 0,1 Mol 4-(3-Chlorphenyl)-1,4-dihydro-6-methyl-pyridazin-3,5-dicarbonsäure-5-ethyl-3-methylester mit 0,105 Mol KOH zu 4-(3-Chlorphenyl)-1,4-dihydro-6-methyl-pyridazin-3,5-dicarbonsäure-5-ethylester verseift. Schmp. 182 °C (Ethanol).

Ausbeute : 89 % d. Th.

Beispiel 31

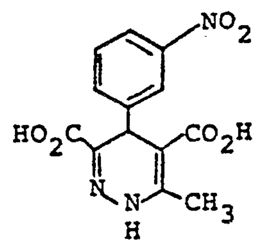

Analog Beispiel 28 wurde 0,1 Mol 1,4-Dihydro-6-methyl-4-(3-nitrophenyl)-pyridazin-3,5-dicarbonsäure-diethylester mit 0,25 Mol KOH zu 1,4-Dihydro-6-methyl-4-(3-nitrophenyl)-pyridazin-3,5-dicarbonsäure verseift. Schmp. 169 °C (Zers).

Ausbeute : 71 % d. Th.

Beispiel 32

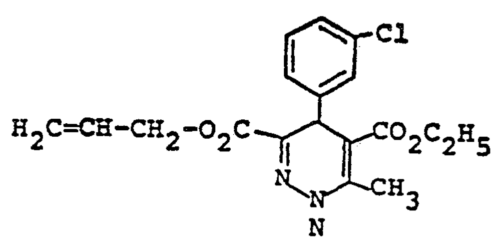

0,1 Mol 4-(3-Chlorphenyl)-1,4-dihydro-6-methyl-pyridazin-3,5-dicarbonsäure-5-ethylester wurde in 150 ml absolutem Allylalkohol unter Zusatz einer Spur p-Toluol-sulfonsäure 18 h am Rückflußkühler erhitzt. Der Alkohol wurde abdestilliert, der Rückstand in Chloroform gelöst und mit Natriumhydrogencarbonat-Lösung gewaschen.

Nach Abdampfen des Chloroforms kristallisierte 4-(3-Chlorphenyl)-1,4-dihydro-6-methylpyridazin-3,5-dicarbonsäure-3-allyl-5-ethylester aus wenig Ethanol, Schmp. 125 °C.

Ausbeute : 82 % d. Th.

Beispiel 33

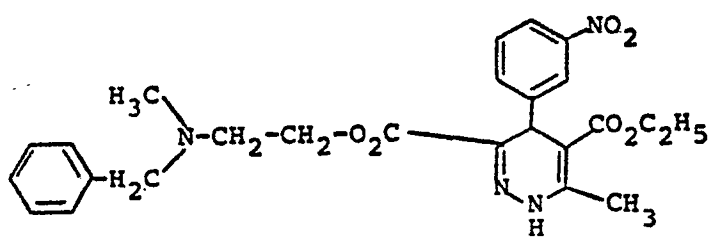

Analog Beispiel 32 wurde aus 0,1 Mol 1,4-Dihydro-6-methyl-4-(3-nitrophenyl)-pyridazin-3,5-dicarbonsäure-5-ethylester nach 20-stündigem Erhitzen in 100 ml β-N-Benzyl-N-methylaminoethanol auf 100 °C unter Zusatz einer Spur p-Toluolsulfonsäure 1,4-Dihydro-6-methyl-4-(3-nitrophenyl)-pyridazin-3,5-dicarbonsäure-3-(β-N-benzyl-N-methylaminoethyl)-5-ethylester vom Schmp. 192 °C (Methanol) erhalten.

Ausbeute : 57 % d. Th.

Beispiel 34

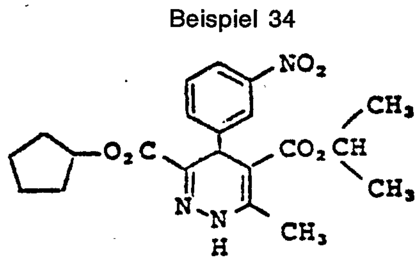

Analog Beispiel 32 wurde aus 0,1 Mol 1,4-Dihydro-6-methyl-4-(2-nitrophenyl)-pyridazin-3,5-dicarbonsäure-5-isopropylester nach 12-stündigem Erhitzen in 100 ml Cyclopentanol auf 100 °C unter Zusatz von 1 ml konz. Schwefelsäure 1,4-Dihydro-6-methyl-4-(3-nitrophenyl)-pyridazin-3,5-dicarbonsäure-3-cyclopentyl-5-isopropylester vom Schmp. 141 °C (aus Methanol nach Kieselgelchromatographie) erhalten.

Ausbeute : 69 % d. Th.

Beispiel 35

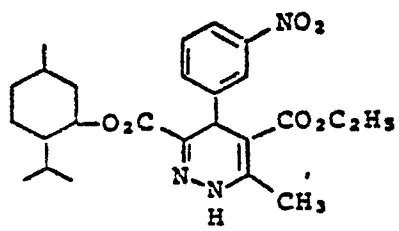

Analog Beispiel 32 wurde 0,1 Mol 1,4-Dihydro-6-methyl-4-(3-nitrophenyl)-pyridazin-3,5-dicarbonsäure-5-ethylester nach 12-stündigem Erhitzen in 30 g l-Menthol auf 100 °C unter Zusatz von 0,5 ml konz. Schwefelsäure 1,4-Dihydro-6-methyl-4-(3-nitrophenyl)-pyridazin-3,5-dicarbonsäure-5-ethyl-3-l-menthylester vom Schmp. 136 °C (Ethanol) erhalten.

Ausbeute : 42 % d. Th.

Beispiel 36

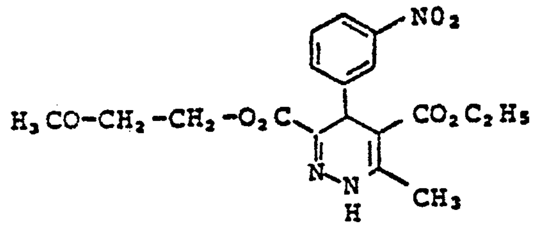

Analog Beispiel 32 wurde aus 0,1 Mol 1,4-Dihydro-6-methyl-4-(3-nitrophenyl)-pyridazin-3,5-dicarbonsäurediethylester wurde nach 12-stündigem Erhitzen in 150 ml siedendem Methoxyethanol unter Zusatz einer Spur p-Toluolsulfonsäure 1,4-Dihydro-6-methyl-4-(3-nitrophenyl)-pyridazin-3,5-dicarbonsäure-5-ethyl-3-(β-methoxyethyl)-ester vom Schmp. 118 °C (Isopropanol) erhalten.

Ausbeute : 79 % d. Th.

Beispiel 37

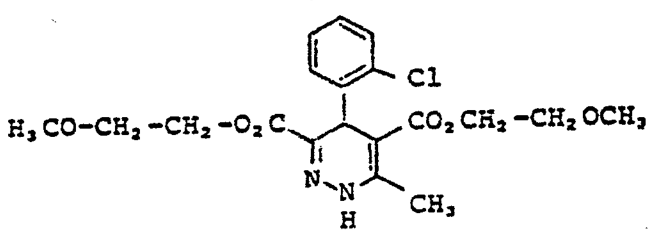

Analog Beispiel 32 wurde aus 0,1 Mol 1,4-Dihydro-6-methyl-4-(3-nitrophenyl)-pyridazin-3,5-dicarbonsäurediethylester wurde nach 48-stündigem Erhitzen in siedendem Methoxyethanol unter Zusatz einer Spur p-Toluolsulfonsäure 4-(2-Chlorphenyl)-1,4-dihydro-6-methyl-pyridazin-3,5-dicarbonsäure-di-(β-methoxyethyl)-ester vom Schmp. 117 °C (aus Isopropanol) erhalten.

Ausbeute : 54 % d. Th.

Beispiel 38

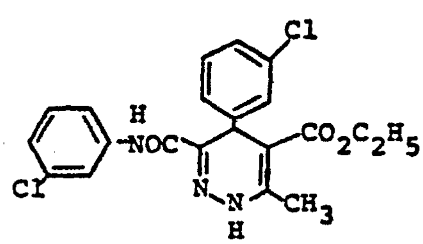

0,05 Mol 4-(3-Chlorphenyl)-1,4-dihydro-6-methyl-pyridazin-3,5-dicarbonsäure-5-ethylester und 0,06 Mol 3-Chloranilin in 50 ml Methylenchlorid wurden unter Eiskühlung mit 0,05 Mol Dicyclohexylcarbodiimid in 20 ml Methylenchlorid versetzt und 12 Std. bei Raumtemperatur gerührt. Der Niederschlag wurde abfiltriert, die organische Phase mit Natriumhydrogencarbonat-Lösung gewaschen, eingeengt und der erhaltene 3-(3-Chlorphenylcarbamoyl)-4-(3-chlorphenyl)-1,4-dihydro-6-methyl-pyridazin-5-carbonsäureethylester aus Ethanol kristallisiert. Schmp. 189 °C.

Ausbeute : 79 % d. Th.

Beispiel 39

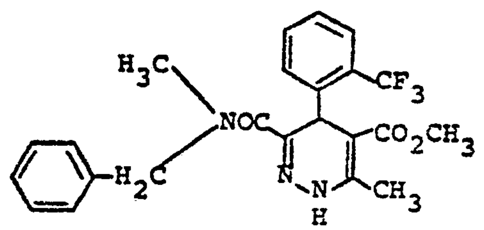

Analog Beispiel 38 wurde durch Umsetzung von 0,05 Mol 1,4-Dihydro-6-methyl-4-(2-trifluormethylphenyl)-pyridazin-3,5-dicarbonsäure-5-methylester mit 0,06 Mol Benzyl-methylamin und 0,05 Mol Dicyclohexylcarbodiimid 3-(Benzyl-methylcarbamoyl)-1,4-Dihydro-6-methyl-4-(2-trifluormethylphenyl)-pyridazin-5-carbonsäuremethylester vom Schmp. 197 °C (Ethanol) erhalten.

Ausbeute : 71 % d. Th.

**Ansprüche**

1. 1,4-Dihydropyridazin-3-carbonylverbindungen der allgemeinen Formel (I)

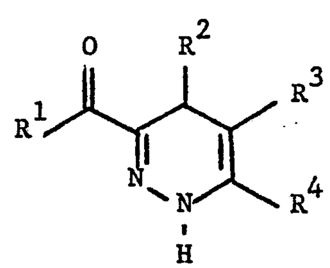

(I)

in welcher

$R^1$ für die Gruppen $OR^5$ oder

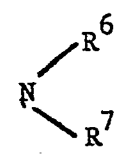

steht, wobei

$R^5$ für Wasserstoff, Allyl, geradkettiges oder verzweigtes Alkyl oder Cycloalkyl mit jeweils bis zu 8 Kohlenstoffatomen, steht, wobei die Alkylkette gegebenenfalls durch 1 Sauerstoff unterbrochen ist oder gegebenenfalls durch eine Aminogruppe mit zwei gleichen oder verschiedenen Substituenten aus der Gruppe Methyl oder Benzyl substituiert ist,

$R^6$ und $R^7$ verschieden sind und jeweils für Wasserstoff, einen Methyl- oder Benzyl- oder einen gegebenenfalls einfach durch Halogen substituierten Phenylrest stehen,

$R^2$ für einen Phenylrest, der gegebenenfalls durch 1 oder 2 gleiche oder verschiedene Substituenten aus der Gruppe Nitro, Halogen, Cyano, Trifluormethyl, Trifluormethoxy, Alkyl oder Alkoxy mit jeweils 1 bis 2 Kohlenstoffatomen substituiert ist oder für einen Thienyl- oder Pyridylrest steht,

$R^3$ für Wasserstoff oder die Grupp $COOR^8$ steht, wobei $R^8$ die Bedeutung von $R^5$ besitzt aber nicht mit $R^5$ identisch sein muß und

$R^4$ für einen Methyl-, Ethyl- oder Phenylrest steht.

2. 1,4-Dihydro-6-methyl-4-(3-nitrophenyl)-pyridazin-3,5-dicarbonsäure-5-ethyl-3-l-menthylester

3. Verfahren zur Herstellung von 1,4-Dihydropyridazin-3-carbonylverbindungen der allgemeinen Formel I

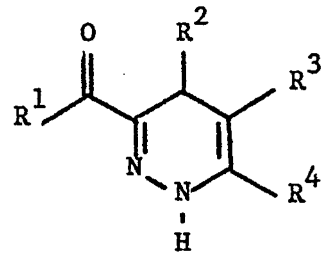

(I)

in welcher

$R^1$ für die Gruppen $OR^5$ oder

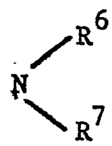

steht, wobei

$R^5$ für Wasserstoff, Allyl, geradkettiges oder verzweigtes Alkyl oder Cycloalkyl mit jeweils bis zu 8 Kohlenstoffatomen, steht, wobei die Alkylkette gegebenenfalls durch 1 Sauerstoff unterbrochen ist oder gegebenenfalls durch eine Aminogruppe mit zwei gleichen oder verschiedenen Substituenten aus der Gruppe Methyl oder Benzyl substituiert ist,

$R^6$ und $R^7$ verschieden sind und jeweils für Wasserstoff, einen Methyl- oder Benzyl- oder einen gegebenenfalls einfach durch Halogen substituierten Phenylrest stehen,

$R^2$ für einen Phenylrest, der gegebenenfalls durch 1 oder 2 gleiche oder verschiedene Substituenten aus der Gruppe Nitro, Halogen, Cyano, Trifluormethyl, Trifluormethoxy, Alkyl oder Alkoxy mit jeweils 1 bis 2 Kohlenstoffatomen substituiert ist oder für einen Thienyl- oder Pyridylrest steht,

$R^3$ für Wasserstoff oder die Gruppe $COOR^8$ steht, wobei $R^8$ die Bedeutung von $R^5$ besitzt aber nicht mit $R^5$ identisch sein muß und

$R^4$ für einen Methyl-, Ethyl- oder Phenylrest steht,

dadurch gekennzeichnet, daß man Nitronsäuren der allgemeinen Formel II

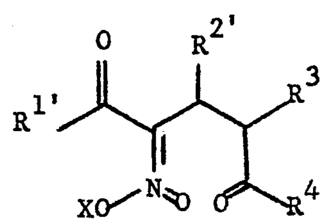

(II)

in welcher

$R^{1'}$ für die Gruppe $OR^{5'}$ steht, wobei $R^{5'}$ für Allyl, geradkettiges oder verzweigtes Alkyl oder Cycloalkyl mit jeweils bis zu 8 Kohlenstoffatomen steht, wobei die Alkylkette gegebenenfalls durch 1 Sauerstoff unterbrochen ist,

$R^{3'}$ für Wasserstoff oder die Gruppe $COR^{8'}$, wobei $R^{8'}$ die oben für $R^8$ angegebene Bedeutung besitzt,

$R^{2'}$ und $R^{4'}$ die oben für $R^2$ und $R^4$ angegebene Bedeutung besitzen, und

X für ein Kation wie Wasserstoff-, Alkali-, Ammonium- oder Alkylamoniumion steht,

zunächst mit Ozon und dann mit Hydrazin bzw. Hydrazinhydrat in inerten organischen Lösungsmitteln umsetzt und gegebenenfalls die so erhaltenen Verbindungen nach allgemein bekannten Methoden der Verseifung, Veresterung, Umesterung oder Amidierung zu weiteren erfindungsgemäßen Verbindungen der allgemeinen Formel I derivatisiert.

4. Verfahren entsprechend Anspruch 3 zur Herstellung von 1,4-Dihydro-6-methyl-4-(3-nitrophenyl)-pyridazin-3,5-dicarbonsäure-5-ethyl-3-l-menthylester.

5. Arzneimittel enthaltend mindestens eine Verbindung gemäß Anspruch 1 und 2.

6. Verfahren zur Herstellung von Arzneimitteln dadurch gekennzeichnet, daß man mindestens eine Verbindung gemäß Anspruch 1 und 2 gegebenenfalls unter Zusatz von geeigneten Hilfs- und Trägerstoffen in eine geeignete Applikationsform überführt.

7. Verbindungen gemäß Anspruch 1 und 2 zur Verwendung bei der Behandlung von Kreislauferkrankungen.

8. Verbindungen gemäß Anspruch 1 und 2 zur Verwendung bei der Behandlung von Spannungszuständen in der glatten Muskulatur.

**Claims**

1. 1,4-Dihydropyridazine-3-carbonyl compounds of the general formula (I)

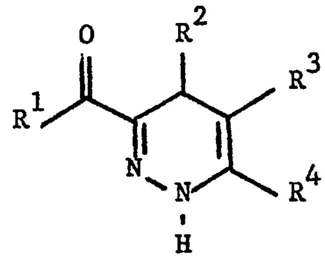

(I)

in which

$R^1$ represents the groups $OR^5$ or

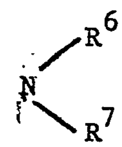

wherein

$R^5$ represents hydrogen, allyl, straight-chain or branched alkyl or cycloalkyl with in each case up to 8 carbon atoms, the alkyl chain optionally being interrupted by 1 oxygen atom or optionally being substituted by an amino group with two identical or different substituents from the group comprising methyl or benzyl.

$R^6$ and $R^7$ are different and in each case represent hydrogen, a methyl or benzyl radical or a phenyl radical which is optionally monosubstituted by halogen,

$R^2$ represents a phenyl radical which is optionally substituted by 1 or 2 identical or different substituents from the group comprising nitro, halogen, cyano, trifluoromethyl, trifluoromethoxy, alkyl or alkoxy with in each case 1 to 2 carbon atoms or represents a thienyl or pyridyl radical,

$R^3$ represents hydrogen or the group $COOR^8$, wherein $R^8$ has the meaning of $R^5$ but must not be identical to $R^5$ and

$R^4$ represents a methyl, ethyl or phenyl radical.

2. 1,4-Dihydro-6-methyl-4-(3-nitrophenyl)-pyridazine-3,5-dicarboxylic acid 5-ethyl 3-l-menthyl ester.

3. Process for the preparation of 1,4-dihydropyridazine-3-carbonyl compounds of the general formula I

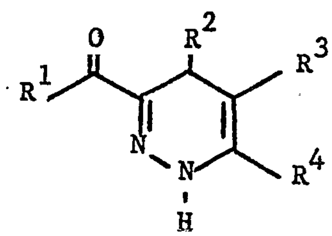

(I)

in which

$R^1$ represents the groups $OR^5$ or

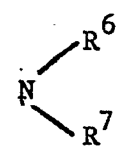

wherein

$R^5$ represents hydrogen, allyl, straight-chain or branched alkyl or cycloalkyl with in each case up to 8 carbon atoms, the alkyl chain optionally being interrupted by 1 oxygen atom or optionally being substituted by an amino group with two identical or different substituents from the group comprising methyl or benzyl,

$R^6$ and $R^7$ are different and in each case represent hydrogen, a methyl or benzyl radical or a phenyl radical which is optionally monosubstituted by halogen,

$R^2$ represents a phenyl radical which is optionally substituted by 1 or 2 identical or different substituents from the group comprising nitro, halogen, cyano, trifluoromethyl, trifluoromethoxy, alkyl or alkoxy with in each case 1 to 2 carbon atoms or represents a thienyl or pyridyl radical,

$R^3$ represents hydrogen or the group $COOR^8$, wherein $R^8$ has the meaning of $R^5$ but must not be identical to $R^5$ and

$R^4$ represents a methyl, ethyl or phenyl radical,

characterised in that nitronic acids of the general formula II

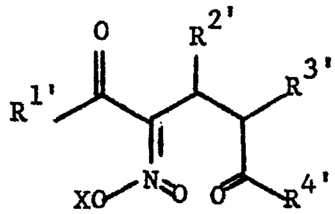

(II)

in which

$R^{1'}$ represents the group $OR^{5'}$, wherein $R^{5'}$ represents allyl, straight-chain or branched alkyl or cycloalkyl with in each case up to 8 carbon atoms, the alkyl chain optionally being interrupted by 1 oxygen atom,

$R^{3'}$ represents hydrogen or the group $COR^{8'}$, wherein $R^{8'}$ has the meaning indicated above for $R^8$, $R^{2'}$ and $R^{4'}$ have the meaning indicated above for $R^2$ and $R^4$, and

X represents a cation such as hydrogen or an alkali metal, ammonium or alkylammonium ion, are first reacted with ozone and then with hydrazine or hydrazine hydrate in inert organic solvents and derivatives are optionally formed from the resulting compounds by generally known saponification, esterification, trans-esterification or amidation methods to give further compounds of the general formula I according to the invention.

4. Process according to Claim 3 for the preparation of 1,4-dihydro-6-methyl-4-(3-nitrophenyl)-pyridazine-3,5-dicarboxylic acid 5-ethyl 3-l-menthyl ester.

5. Medicaments containing at least one compound according to Claim 1 and 2.

6. Process for the preparation of medicaments, characterised in that at least one compound according to Claim 1 and 2 is converted into a suitable form of administration, suitable auxiliaries and excipients being added if appropriate.

7. Compounds according to Claim 1 and 2 for use in the treatment of circulatory diseases.

8. Compounds according to Claim 1 and 2 for use in the treatment of tonicities in smooth muscle.

**Revendications**

1. Composés de 1,4-dihydropyridazine 3-carbonyles de formule générale (I)

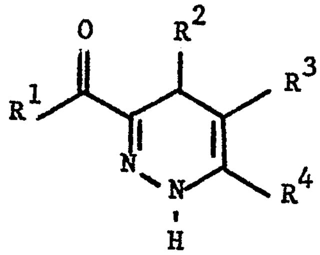

(I)

dans laquelle

$R^1$ représente les groupes $OR^5$ ou

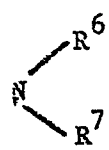

où

$R^5$ est un atome d'hydrogène ou un groupe allyle, alkyle linéaire ou ramifié ou cycloalkyle, ayant jusqu'à 8 atomes de carbone, la chaîne alkyle pouvant éventuellement être interrompue par un atome d'oxygène ou être éventuellement substituée par un groupe amino comportant deux substituants, identiques ou différents, choisis parmi un groupe méthyle et un groupe benzyle,

$R^6$ et $R^7$ sont différents et représentent chacun un atome d'hydrogène, un radical méthyle ou benzyle ou un radical phényle éventuellement substitué une fois par un halogène,

$R^2$ est un radical phényle, éventuellement substitué par un ou deux substituants, identiques ou différents, choisis parmi un groupe nitro, un atome d'halogène, un groupe cyano, trifluorométhyle, trifluorométhoxy, alkyle ou alcoxy ayant chaque fois un ou deux atomes de carbone, ou $R^2$ est un radical thiényle ou pyridyle,

$R^3$ est un atome d'hydrogène ou représente le groupe $COOR^8$, où $R^8$ a le sens de $R^5$ mais ne doit pas être identique à $R^5$, et

$R^4$ est un radical méthyle, éthyle ou phényle.

2. 1,4-Dihydro 6-méthyl 4-(3-nitrophényl) pyridazine 3,5-dicarboxylate de 5-éthyle et de 3-1-mentyle.

3. Procédé de production de composés de 1,4-dihydropyridazine 3-carbonyles de formule générale (I)

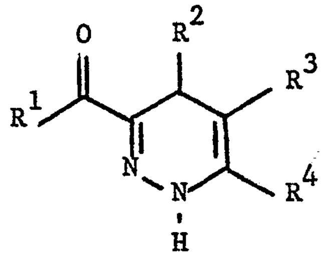

(I)

dans laquelle

$R^1$ représente les groupes $OR^5$ ou

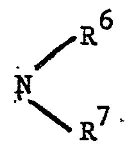

où

$R^5$ est un atome d'hydrogène ou un radical allyle, alkyle linéaire ou ramifié ou cycloalkyle ayant jusqu'à 8 atomes de carbone, la chaîne alkyle étant éventuellement interrompue par un atome d'oxygène ou étant éventuellement substituée par un groupe amino comportant deux substituants, identiques ou différents, choisis parmi un groupe méthyle et un groupe benzyle,

$R^6$ et $R^7$ sont différents et représentent chacun un atome d'hydrogène, un radical méthyle ou benzyle ou un radical phényle éventuellement substitué une fois par un halogène,

$R^2$ est un radical phényle, pouvant éventuellement être substitué par un ou deux substituants, identiques ou différents, choisis parmi un radical nitro, halogène, cyano, trifluorométhyle, trifluorométhoxy, alkyle ou alcoxy ayant chacun un à deux atomes de carbone, ou bien $R^2$ est un radical thiényle ou pyridyle,

$R^3$ est un atome d'hydrogène ou représente le groupe $COOR^8$, dans lequel $R^8$ a le sens de $R^5$ mais ne doit pas être identique à $R^5$, et

$R^4$ est un radical méthyle, éthyle ou phényle,

caractérisé en ce qu'on fait réagir des acides acinitrés de formule générale (II)

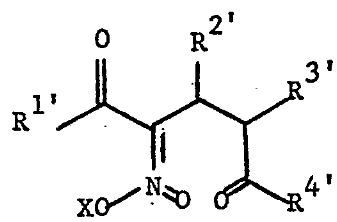

(II)

dans laquelle

$R^{1'}$ représente le groupe $OR^{5'}$, où $R^{5'}$ est un radical allyle, alkyle linéaire ou ramifié ou cycloalkyle ayant chacun jusqu'à 8 atomes de carbone, la chaîne alkyle étant éventuellement interrompue par un atome d'oxygène,

$R^{3'}$ est un atome d'hydrogène ou est le groupe $COR^{8'}$, où $R^{8'}$ a le sens indiqué ci-dessus pour $R^8$,

$R^{2'}$ et $R^{4'}$ ont le sens indiqué ci-dessus pour $R^2$ et $R^4$, et

X est un cation comme un atome d'hydrogène ou un métal alcalin ou un ion ammonium ou alkylammonium,

tout d'abord avec l'ozone puis avec l'hydrazine ou l'hydrate d'hydrazine dans des solvants organiques inertes et en ce qu'on transforme éventuellement les composés ainsi obtenus, selon des procédés généraux connus de saponification, estérification, tranestérification ou amidation, pour obtenir des dérivés qui sont d'autres composés de l'invention répondant à la formule générale (I).

4. Procédé selon la revendication 3 pour produire le 1,4-dihydro 6-méthyl 4-(3-nitrophényl) pyridazine 3,5-dicarboxylate de 5-éthyle et de 3-1-mentyle.

5. Médicaments contenant au moins un composé selon la revendication 1 ou 2.

6. Procédé de production de médicaments, caractérisé en ce qu'on transforme au moins un composé selon les revendications 1 et 2, éventuellement avec addition d'adjuvants et véhicules ou excipients convenables, en une forme convenant pour l'application ou l'administration.

7. Composés selon les revendications 1 et 2, destinés à être utilisés pour le traitement des maladies de la circulation du sang.

8. Composés selon les revendications 1 et 2, destinés à être utilisés pour le traitement des états de la tonicité des muscles lisses.